# EUROPEAN PATENT APPLICATION

(11) **EP 4 834 244 A1**
(43) Date of publication of application: **04.11.2026**
(21) Application number: 24911245.9
(22) Date of filing: 25.12.2024
(51) Int. Cl.: A61B 17/12

(54) **OCCLUSION DEVICE**

(30) Priority: 29.12.2023 CN 202311868121
(71) Applicant: Lifetech Scientific (Shenzhen) Co., Ltd., Shenzhen, Guangdong 518063 (CN)
(72) Inventor: LIU, Jianyong, Shenzhen, Guangdong 518063 (CN); ZHENG, Xuxuan, Shenzhen, Guangdong 518063 (CN); CAO, Xiaowei, Shenzhen, Guangdong 518063 (CN)
(74) Representative: Michalski Hüttermann & Partner mbB
(86) International application number: PCT/CN2024/142349
(87) International publication number: WO 2025/140322

(57) **Abstract**

The present application relates to an occlusion device, including a fixing portion and a sealing portion. The fixing portion includes at least one support member. The occlusion device has an initial state and an assembled state. In the initial state, a first connection portion and a second connection portion are separated from each other. In the assembled state, the first connection portion is connected to the second connection portion to connect the fixing portion to the sealing portion. Therefore, the sealing portion and the fixing portion can be relatively independently packaged and produced, and assembled according to required sizes when implantation is required. Thus, an assembled left atrial appendage occlusion device exhibits good adaptability and can also adapt to shapes of some left atrial appendages with special sizes.

## Description

This application claims priority to Chinese Patent Application No. 202311868121.3, filed with China National Intellectual Property Administration on December 29, 2023 and entitled "Occlusion Device".

### Technical Field

The present application relates to the technical field of interventional medical devices, and in particular, to an occlusion device.

### Background Art

In interventional medicine, an occlusion device is an instrument for closing or occluding a blood vessel or a cavity, and is usually configured to treat vascular abnormalities or lesions, to block abnormal blood flow or close abnormal channels. Common occlusion devices mainly include a vascular occlusion device, an orifice occlusion device, and a catheter occlusion device. The orifice occlusion device is mainly configured to close pores or defects in a heart, such as a left atrial appendage, an atrial septal defect, or patent ductus arteriosus. The left atrial appendage is taken as an example. The left atrial appendage is a long and narrow, curved blind-end structure that extends forwards and downwards along the anterolateral wall of a left atrium, and has active systolic and diastolic functions and a secretion function. Cerebral apoplexy caused by atrial fibrillation mainly originates from the left atrial appendage. During interventional surgery, a left atrial appendage occlusion device can usually be used to occlude the orifice of the left atrial appendage to prevent thrombus from entering the left atrium and causing cerebral apoplexy caused by atrial fibrillation, and to block blood flow from the left atrium into the left atrial appendage.

At present, there are mainly two types of left atrial appendage occlusion devices on the market: a plug type left atrial appendage occlusion device and a double-disc type left atrial appendage occlusion device. The left atrial appendage occlusion device with a double-disc structure usually includes a fixing disc and a sealing disc. In an existing disc type left atrial appendage occlusion device, the fixing disc and the sealing disc are usually fixedly connected as a whole through welding or an intermediate connection member.

However, the fixing disc and the sealing disc of the left atrial appendage occlusion device implanted into the body of a patient generally need to be fitted according to the shape, size, etc. of a corresponding position of the left atrial appendage of the patient. The specifications of the existing left atrial appendage occlusion devices are limited and satisfy relative size limitations, and the size and shape of the left atrial appendage will only be accurately measured during the implantation stage of the procedure. Without a proper left atrial appendage occlusion device, the surgery cannot be carried out. Customizing a new left atrial appendage occlusion device would necessitate a re-implantation procedure, which poses a significant risk.

### Summary of the Invention

Based on this, it is necessary to provide an improved occlusion device to solve the technical problems that the existing occlusion device is of an integrated structure, so that excessive reserves increase the stock and the difficulty of production and management, and insufficient reserves delay the operation timing, specifically as follows:
An occlusion device is provided, including a fixing portion and a sealing portion. The fixing portion includes at least one support member. The occlusion device has an initial state and an assembled state. In the initial state, a first connection portion and a second connection portion are separated from each other. In the assembled state, the first connection portion is connected to the second connection portion to connect the fixing portion to the sealing portion.

In one embodiment, the fixing portion and the sealing portion are stably connected after being assembled.

In one embodiment, the first connection portion includes a connector that extends out in an axial direction; the connector includes a main body and a stop piece which are distributed in an extended manner from a proximal end to a distal end; and the diameter of the stop piece is greater than the diameter of the main body.

In one embodiment, the second connection portion includes a hollow tube body structure; and the second connection portion includes a plurality of stop sheets that obliquely extend out from the tube body structure towards an inner side and the distal end and are oppositely arranged in a circumferential direction.

In one embodiment, the inner diameter of the tube body structure is greater than the outer diameter of the connector; and the inner diameter of a channel enclosed by free ends of the plurality of stop sheets is less than the outer diameter of each stop sheet.

In one embodiment, the diameter of the stop piece gradually decreases from the proximal end to the distal end.

In one embodiment, a step surface is arranged at a connection position or a transition region between the stop piece and the main body.

In one embodiment, the first connection portion includes at least one positioning member distributed in a circumferential direction; and at least one positioning slot that matches the positioning member is arranged at the proximal end of the second connection portion.

In one embodiment, a proximal end surface or a distal end surface of the sealing portion includes a straight section and a curved section; the curved section is located on an outer side of the straight section; and an edge of the curved section is located at a distal end of the straight section.

In one embodiment, in a natural state, the length of the curved section from a proximal end to a distal end is 1 to 5 mm.

Compared with the prior art, the present application provides an occlusion device, including a fixing portion and a sealing portion. The fixing portion includes at least one support member. The occlusion device has an initial state and an assembled state. In the initial state, a first connection portion and a second connection portion are separated from each other. In the assembled state, the first connection portion is connected to the second connection portion to connect the fixing portion to the sealing portion. Therefore, the sealing portion and the fixing portion can be relatively independently packaged and produced, and assembled according to required sizes when implantation is required. Thus, an assembled left atrial appendage occlusion device exhibits good adaptability and can also adapt to the shapes of some left atrial appendages with special sizes.

### Brief Description of the Drawings

FIG. 1 is a schematic structural diagram of an occlusion device in a natural state in one embodiment of the present application;
FIG. 2 is a schematic external structural diagram of an occlusion device in a natural state in one embodiment of the present application;
FIG. 3 is a schematic structural diagram of a front-view state of a fixing portion of an occlusion device in a natural state in one embodiment of the present application;
FIG. 4 is a schematic structural diagram of a top-view state of a fixing portion of an occlusion device in a natural state in one embodiment of the present application;
FIG. 4a is a schematic structural diagram of a top-view state of a first variant of a fixing portion of an occlusion device in a natural state in one embodiment of the present application;
FIG. 4b is a schematic structural diagram of a top-view state of a second variant of a fixing portion of an occlusion device in a natural state in one embodiment of the present application;
FIG. 4c is a schematic structural diagram of a top-view state of a third variant of a fixing portion of an occlusion device in a natural state in one embodiment of the present application;
FIG. 5 is a schematic structural diagram of a sealing portion of an occlusion device in a natural state in one embodiment of the present application;
FIG. 6 is a schematic structural diagram of an occlusion device before assembling according to one embodiment of the present application;
FIG. 7 is a schematic diagram of an assembled part of an occlusion device in one embodiment of the present application;
FIG. 8 is a schematic structural diagram of a first fixture for assembling of an occlusion device in one embodiment of the present application;
FIG. 9 is a schematic diagram of a first state of a first fixture for assembling of an occlusion device in one embodiment of the present application;
FIG. 10 is a schematic diagram of a second state of a first fixture for assembling of an occlusion device in one embodiment of the present application;
FIG. 11 is a schematic structural diagram of a second fixture for assembling of an occlusion device in one embodiment of the present application;
FIG. 12 is a schematic diagram of operation of one clamp of a second fixture for assembling of an occlusion device in one embodiment of the present application;
FIG. 13 is a schematic diagram of operation of another clamp of a second fixture for assembling of an occlusion device in one embodiment of the present application;
FIG. 14 is a schematic structural diagram of a blocking member in one embodiment of the present application;
FIG. 15 is a schematic diagram of the operation of a second fixture for assembling of an occlusion device in one embodiment of the present application;
FIG. 16 is a schematic structural diagram of an occlusion device in another embodiment of the present application;
FIG. 17 is a schematic diagram of the connection of a fixing portion and a sealing portion of an occlusion device in another embodiment of the present application;
FIG. 18 is a schematic structural diagram of an occlusion device in another embodiment of the present application;
FIG. 19 is a schematic diagram of connection of a fixing portion and a sealing portion of an occlusion device in another embodiment of the present application; and
FIG. 20 is a schematic diagram of connection of a fixing portion and a sealing portion of an occlusion device in another embodiment of the present application.

### Detailed Description of the Invention

To make the objectives, technical solutions, and advantages of the present application clearer, the following further describes the present application in detail with reference to the accompanying drawings and the embodiments. It should be understood that the specific embodiments described herein are merely used to explain the present application but are not intended to limit the present application.

It should be noted that in the field of interventional medical devices, an end, closer to an operator, of a medical device implanted in a human or animal body is generally referred to as a "proximal end", and an end that is farther away from the operator is referred to as a "distal end". Based on this principle, the "proximal end" and "distal end" of any component of the medical device are defined. "Axial direction" means a length direction of a medical instrument during delivery. "Radial direction" generally means a direction which is perpendicular to the "axial direction" of the medical instrument. Based on this principle, an "axial direction" and a "radial direction" of any component of the medical instrument are defined. The "connection" mentioned in the embodiments includes situations in which two assemblies are directly connected and indirectly connected through other assemblies.

The following will further explain the technical solutions of the present application in detail in conjunction with specific embodiments.

An occlusion device provided by the embodiments of the present application can be configured to occlude a left atrial appendage or can be configured to occlude other internal tissues with orifices, including and not limited to atrial septal defects, patent ductus arteriosus, and other symptoms. An example in which a left atrial appendage is occluded is used for describing the occlusion device in detail.

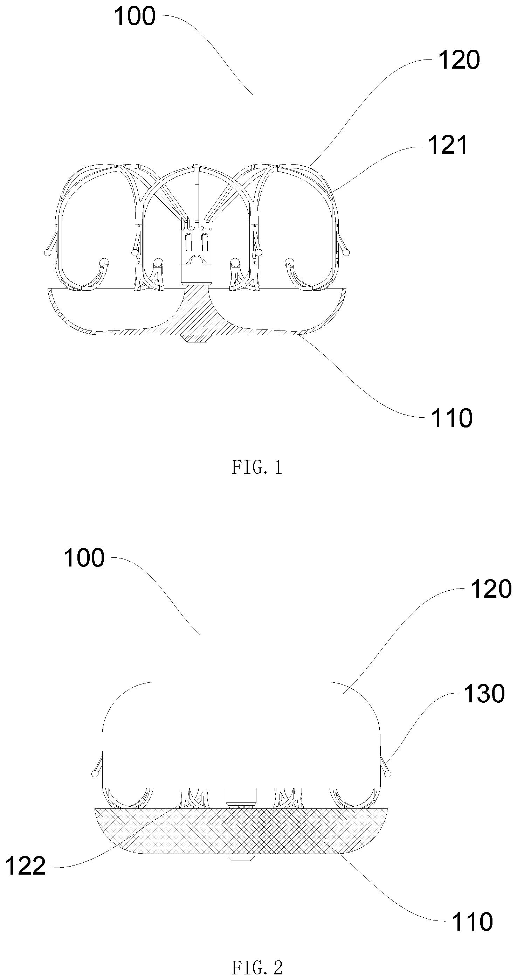

Referring to FIG. 1 to FIG. 2, FIG. 1 is a schematic structural diagram of an occlusion device 100 in a natural state in one embodiment of the present application. FIG. 2 is a schematic external structural diagram of an occlusion device 100 in a natural state in one embodiment of the present application. The occlusion device 100 includes a fixing portion 120 and a sealing portion 110 connected to the fixing portion 120. The sealing portion 110 and the fixing portion 120 are spaced apart in an axial direction of the occlusion device 100. The sealing portion 110 is located at a proximal end of the occlusion device 100, and the fixing portion 120 is located at a distal end of the occlusion device 100. The occlusion device 100 has a compressed state in which it is received within a sheath for easy delivery, and an expanded state in which it extends out from a distal end of the sheath and self-expands as shown in FIG. 1. The configuration of the occlusion device 100 after it is released in a cavity of the left atrial appendage is exactly the same as or generally consistent with that in FIG. 1. In other embodiments, for example, when the occlusion device is applied to occluding an atrial septal defect, the sealing portion 110 and the fixing portion 120 can approach each other after release, to fix the occlusion device 100 in a septum between a left atrium and a right atrium.

In this embodiment, the sealing portion 110 is braided into a mesh tube by a plurality of braiding wires 111. At two ends of the mesh tube, end portions of the braiding wires 111 are closed and fixed by sleeves. Afterwards, the mesh tube is subjected to heat setting into a disc, a column, or a plug to obtain the sealing portion 110 for sealing an orifice of the left atrial appendage. The sealing portion 110 includes a distal disc surface 112 facing the fixing portion 120, and a proximal disc surface 113 opposite to the distal disc surface 112. At least one layer of thin film body (not shown) used as a flow blocking film is arranged inside the sealing portion 110. An edge of the thin film body is fixed on a braiding wire 111 at an edge of the sealing portion 110 by usually using a suture, and the thin film body is located between the distal disc surface 112 and the proximal disc surface 113.

In another embodiment, the thin film body is clung to the proximal disc surface 113 or the distal disc surface 112. When a plurality of thin film bodies are provided, positions of the thin film bodies can be arbitrarily selected.

The thin film bodies are configured to prevent a blood flow from flowing from one side of the sealing portion 110 to another side, so as to prevent the blood flow from flowing between the left atrial appendage and the left atrium.

In another embodiment, the total number of strands of braiding wires 111 used in the sealing portion 110 increases. In one aspect, more braiding wires can objectively better block the blood flow and support the flow blocking film, thereby achieving a better sealing effect. In another aspect, an edge of a disc surface of the sealing portion 110 formed by braiding is braided by more wires to further fill gaps at the edge, thereby obtaining a smoother disc surface edge and making it more closely attached to an inner wall of the left atrial appendage compared with a traditional setting. In another embodiment, the total number of braiding wires 111 exceeds 72, which means that the sealing frame of the sealing portion 110 is of a dense mesh structure, preferably 114 strands of wires.

In another embodiment, when the sealing frame of the sealing portion 110 is of the dense mesh structure, the meshed structure formed by braiding of the braiding wires 111 has already been dense enough to block outflow of thrombi in the atrial appendage, thus eliminating the flow blocking film.

The fixing portion 120 includes at least one support member 121 and a lamination film that covers a surface of the support member 121, and the sealing portion 110 is directly or indirectly connected to the fixing portion 120. The support member 121 on the fixing portion 120 can be a rod obtained by cutting a metal alloy tube or a polymer tube, or a rod made by weaving or winding braiding wires. In this embodiment, the specific shape of the support member 121 is not limited. In order to improve the anchoring capability of the fixing portion 120, in this embodiment, an anchoring member 130 is arranged on the fixing portion 120. The anchoring member 130 can be a structure such as an anchoring thorn or a ball head that can be inserted into a predetermined position.

In this embodiment, the support member 121 of the fixing portion 120 is not necessarily a completely independent support rod structure, or there may be crosslinking between adjacent support members 121, as long as the whole can play a supporting role. A plurality of support members 121 extend from a central convergence position towards the distal end, and extend towards the proximal end after being everted.

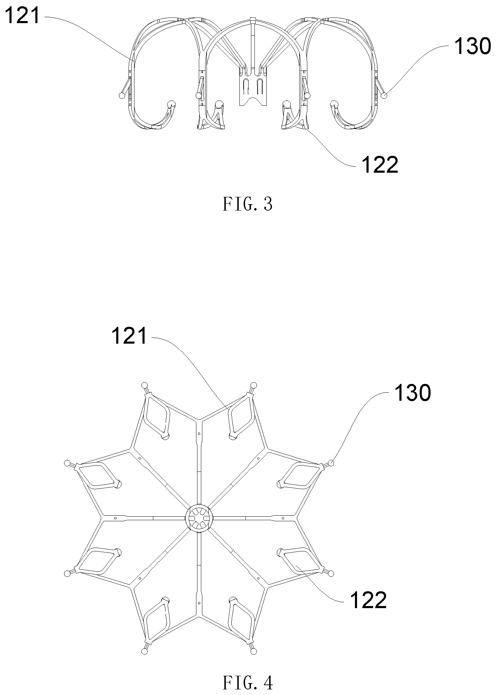

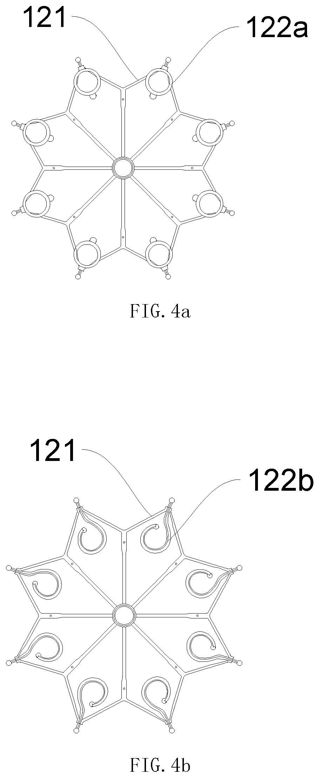

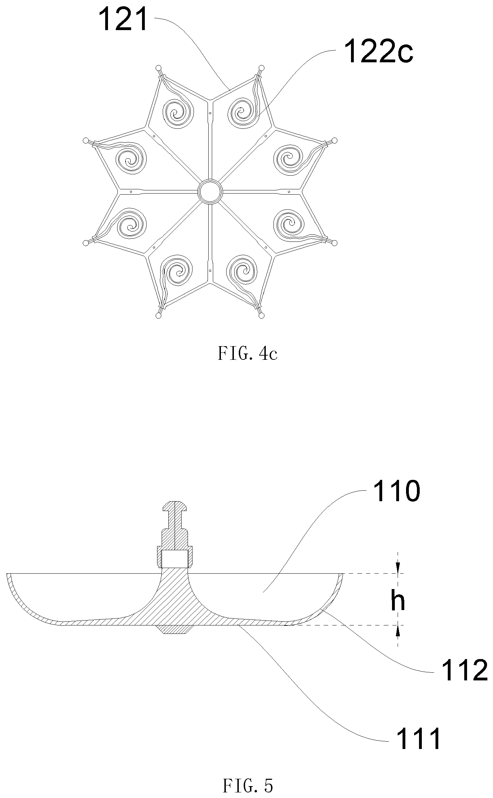

In this embodiment, further referring to FIG. 3 to FIG. 4, FIG. 3 is a schematic structural diagram of a front-view state of a fixing portion 120 of an occlusion device 100 in a natural state in one embodiment of the present application. FIG. 4 is a schematic structural diagram of a top-view state of a fixing portion 120 of an occlusion device 100 in a natural state in one embodiment of the present application. For a single support member 121, a blocking member 122 is arranged at a proximal end of the support member 121. The blocking member 122 can be directly cut from an end of the support member 121 (that is, it is integrally formed with the support member 121 or belongs to a part of the support member 121), or fixed to the proximal end of the support member 121 in various manners such as welding and bonding.

In a natural state, the circumferential width of the blocking member 122 is greater than the width of a portion of the support member 121 that is close to the blocking member 122. For ease of quantification, starting from a root portion of the anchoring member 130, the position of the support member 121 from the root portion of the anchoring member 130 to the most proximal end of the support member 121 is recorded as an extension section of the support member 121. The circumferential width of the blocking member 122 is greater than the width of the extension section of the support member 121, so that when the fixing portion 120 approaches the sealing portion 110, the blocking member 122 can prevent the proximal end of the support member 121 from being stuck in the sealing portion 110 or lower the risk that the proximal end of the support member 121 is stuck in the sealing portion 110.

In this embodiment, to avoid the impact of the blocking member 122 on smooth entry and exit of the entire occlusion device 100 into the sheath, the blocking member 122 is preferably a hollow annular structure. The annular shape here is not strictly a circular ring shape, but means that the blocking member 122 is a closed ring with closed ends, that is, the blocking member 122 does not have an opening.

In another embodiment, the blocking member 122 includes at least a pair of connection rods partially away from each other. The blocking member 122 extends out of the extension section of the support member 121 and is then split into a pair of connection rods. A gap exists between at least some positions of the two connection rods, and ends of the two connection rods are close to and connected to each other. The ends that are close to and connected to each other are smooth and are preferably spherical, to avoid the risk of potential damage to an implantation site (such as the inner wall of the atrial appendage).

Preferably, in this embodiment, the blocking member 122 extends from the extension section of the support member 121 towards the distal end to form a U-shaped structure that is fitted to the extension section of the support member 121 as a whole, so that a free end of the fixing portion 120 is away from the implantation site (such as the inner wall of the atrial appendage), thereby avoiding stress concentration or damage to the implantation site.

For the prior art, if the blocking member 122 as in this embodiment is not designed, it is extremely easy for the extension section of the support member 121 (or the end of the support member 121) to get stuck into the sealing portion 110. Therefore, in the prior art, a distance is generally reserved between the fixing portion 120 and the sealing portion 110, so that the entire occlusion device 100 has a large length. Since the left atrial appendage contracts and relaxes as the heart contracts and relaxes, the prior art always has a high risk that the extension section of the support member 121 (or the end of the support member 121) gets stuck into the sealing portion 110. Therefore, the design of the blocking member 122 in this embodiment can significantly reduce or even prevent the extension section of the support member 121 (or the end of the support member 121) from being stuck in a braided body of the sealing portion 110.

In another embodiment, the circumferential width of the blocking member 122 is greater than widths (also understood as diameters) of grids of all distal end surfaces of the braided body of the sealing portion 110, so that the blocking member 122 cannot be directly stuck in the sealing portion 110.

In another embodiment, the blocking member 122 radially spans at least two grids of a distal end surface of the sealing portion 110, thereby preventing the blocking member 122 from being directly stuck in the sealing portion 110.

In another embodiment, the blocking member 122 circumferentially spans at least two grids of a distal end surface of the sealing portion 110, thereby preventing the blocking member 122 from being directly stuck in the sealing portion 110.

In another embodiment, in the natural state, the entire blocking member 122 is located at a proximal end of the anchoring member 130.

In another embodiment, the circumferential width of the blocking member 122 is greater than twice the width of the largest grid of a distal end surface of the braided body of the sealing portion 110, so that the blocking member 122 cannot be directly stuck in the sealing portion 110 even if the sealing portion deforms.

In another embodiment, the blocking member 122 is in another shape. Referring to FIG. 4a, FIG. 4b, and FIG. 4c. FIG. 4a is a schematic structural diagram of a top-view state of a first variant of a fixing portion of an occlusion device in a natural state in one embodiment of the present application; FIG. 4b is a schematic structural diagram of a top-view state of a second variant of a fixing portion of an occlusion device in a natural state in another embodiment of the present application; and FIG. 4c is a schematic structural diagram of a top-view state of a third variant of a fixing portion of an occlusion device in a natural state in yet another embodiment of the present application. Based on the above figures together, the blocking member 122 can be designed as a circular-ring-shaped blocking member 122a. The blocking member 122a is smoother and less likely to be stuck in a sealing portion 110 than the foregoing blocking member 122. The blocking member 122b can be set as a single blocking rod, and a projection of the blocking member 122b on a cross section of the fixing portion is arc-shaped. A free end of the blocking member 122b is located on a distal end of a starting end, and the projection of the blocking member 122b on the cross section of the fixing portion is a major arc, so that the proximal end of the blocking member 122b is continuous, thus avoiding the free end from being stuck in the sealing portion 110. The blocking member 122c can be configured as a single rod. A projection of the blocking member 122c on the cross section of the fixing portion is at least one spiral circumference. A free end of the blocking member 122c is located on a distal end of a starting end, which can prevent the free end from being stuck in the sealing portion 110. It should be emphasized that although the blocking member 122b and the blocking member 122c are not closed structures, it is easier to manufacture and shape the blocking member 122b and the blocking member 122c, and the costs are lower.

In this embodiment, the sealing portion 110 is also optimized. Further referring to FIG. 5, FIG. 5 is a schematic structural diagram of a sealing portion 110 of an occlusion device 100 in a natural state in one embodiment of the present application. In terms of the overall shape, an edge of the sealing portion 110 is located at the most distal end of the entire sealing portion 110, so that the entire sealing portion 110 is in a state of protruding towards the proximal end.

Specifically, in this embodiment, a proximal end surface and a distal end surface of the sealing portion 110 are tightly abutted (in this embodiment, the tight abutment in the present embodiment does not refer to constant contact in the strict sense. Tight abutment shall be deemed to exist if the distance between them is close to zero or the gap is less than 1 mm.). The proximal end surface of the sealing portion 110 includes a straight section 111 and a curved section 112. The curved section 112 is located on an outer side of the straight section 111, and an edge of the curved section 112 is located at a distal end of the straight section 111. In the natural state, a length h of the curved section 112 from the proximal end to the distal end is 1 to 5 mm, preferably 3 mm.

Therefore, due to the presence of the curved section 112, the sealing portion 110 has a relatively larger axial length compared with an ordinary disc-shaped structure. It can even partially overlap the fixing portion 120 in the axial direction without being in contact with the fixing portion 120, thereby overall shortening the axial length of the entire occlusion device 100, which is more conducive to reducing occurrence of rejection reactions. Due to the arrangement of the foregoing fixing portion 120, the axial length of the occlusion device 100 can be shortened.

In addition, since the edge of the sealing portion 110 is located at the most distal end of the entire sealing portion 110, the sealing portion 110 can be used as a cover to cover the orifice of the auricular appendage or inserted into the auricular appendage. The compression on the auricular appendage can cause the outer side edge of the sealing portion 110 to have an axial length, thereby enlarging the contact area between the sealing portion 110 and the inner wall of the auricular appendage and enhancing the sealing effect of the sealing portion 110. Therefore, when the auricular appendage is deeper, the manner of inserting the sealing portion into the auricular appendage can be selected. When the auricular appendage is shallower, the sealing portion can cover the orifice of the auricular appendage, thus greatly improving the adaptability of the occlusion device 100 to environments. Most importantly, due to the above design, the edge located on the proximal end of the occlusion device 100 is tightly abutted to the inner wall or orifice of the atrial appendage after implantation, so that no new thrombus will be formed due to a gap between the edge and the inner wall or orifice of the atrial appendage.

It should be noted that when the length h of the curved section 112 from the proximal end to the distal end is greater than 5 mm, the axial length of the curved section 112 may be too large, which affects the anchoring of the anchoring member 130 and possibly interfere with and abrade the lamination film on the fixing portion 120. When the length h of the curved section 112 from the proximal end to the distal end is less than 1 mm, the curved section 112 is prone to bulge towards the distal end when compressed, thereby achieving a completely opposite technical effect to this embodiment and affecting good sealing of the occlusion device 100. The length of the curved section 112 from the proximal end to the distal end should be as close as possible to 3 mm. Considering a process error, the length of the curved section 112 from the proximal end to the distal end can be preferably 2 to 4 mm.

In another embodiment, the proximal end surface and the distal end surface of the sealing portion 110 are not completely tightly attached, and the above effect depends more on the distal end surface. Therefore, in this embodiment, a straight section and a curved section are correspondingly arranged on the distal end surface of the sealing portion, so that a length of the curved section from the proximal end to the distal end is 1 to 5 mm, preferably 3 mm, and other settings are the same as those in the foregoing embodiment.

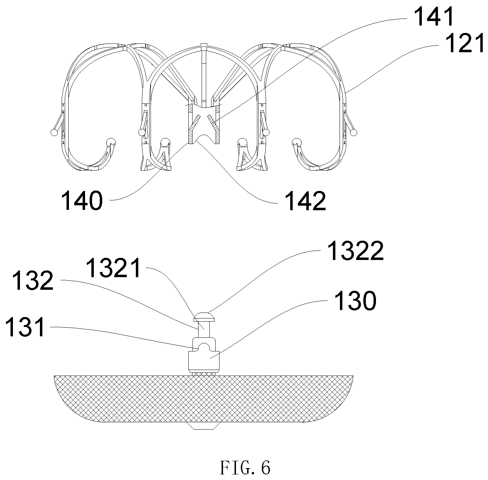

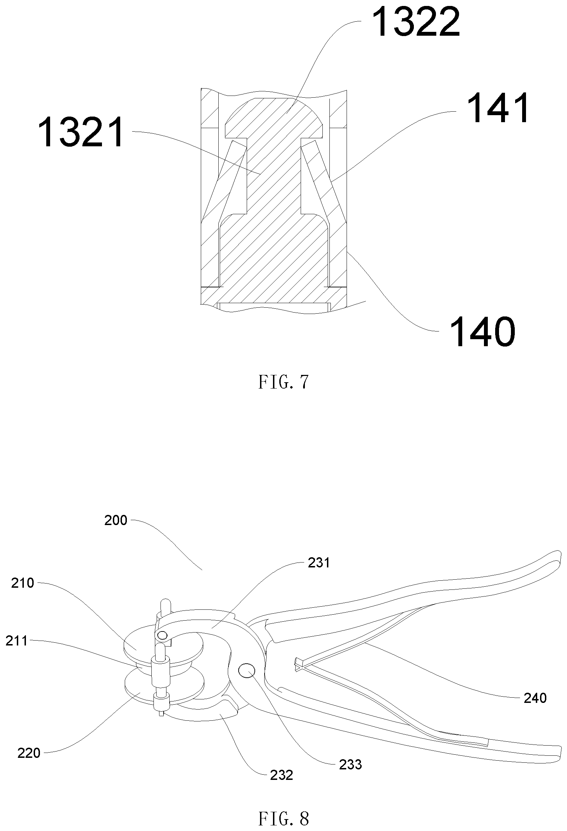

Referring to FIG. 6 to FIG. 7, FIG. 6 is a schematic structural diagram of an occlusion device 100 before assembling in one embodiment of the present application. FIG. 7 is a schematic diagram of an assembled part of an occlusion device 100 in one embodiment of the present application. In this embodiment, the sealing portion 110 and the fixing portion 120 are relatively independent before use. That is, the sealing portion 110 and the fixing portion 120 are assembled during use. Therefore, the sealing portion 110 and the fixing portion 120 can be transported and manufactured independently, thus reducing production procedures and transportation costs. In addition to manufacturing and transportation, more importantly, in left atrial appendage occlusion surgery, an operator needs to first introduce a sheath and measure the size of the atrial appendage during implantation, and then implant an occlusion device with a proper size based on a measurement result. That is, the special feature of left atrial appendage occlusion surgery is that the selection process of a left atrial appendage occlusion device is essentially one of surgical steps. Therefore, since the size required by a user cannot be accurately determined, the operator needs to prepare various or all specifications of left atrial appendage occlusion devices in advance, which undoubtedly increases the costs significantly. In addition, it should be noted that sizes of parts of the left atrial appendage occlusion device in the prior art are fixed after the manufacturing. That is, corresponding specifications have been determined before the operator uses the occlusion device. Manufacturers manufacture left atrial appendage occlusion devices of various specifications to meet requirements of common atrial appendage shapes or sizes. However, the size of the atrial appendage of each user cannot be predicted in advance, and the common specifications in the prior art only vary in sizes (for example, the diameter of the fixing portion of the left atrial appendage occlusion device of a common specification is less than the diameter of the sealing portion by about 6 to 10 mm), so that if the size of the atrial appendage of the user cannot be perfectly fitted, only a left atrial appendage occlusion device of a larger specification can be implanted. Most importantly, if the size of the atrial appendage of a patient is special (for example, the sizes of the orifices of the atrial appendages of some patients are basically the same as internal sizes of the atrial appendages, or the sizes of the orifices of the atrial appendages are smaller than the internal sizes of the atrial appendages, so the diameters of the required fixing portion and sealing portion are close to or even larger), this is beyond the common preset specifications, and the user can only choose to stop the implantation procedure. The puncture and other steps before the implantation undoubtedly become injury to the user. If another left atrial appendage occlusion device is customized later, the surgery needs to be carried out again after the customization. This brings a significant risk. Therefore, in the prior art, the size of the left atrial appendage occlusion device often cannot perfectly match the atrial appendage, and the sizes of the atrial appendages of some patients exceed the specifications of the conventional left atrial appendage occlusion devices. Therefore, the design in which the sealing portion 110 and the fixing portion 120 can be assembled in this embodiment is used. After the operator measures the size of the atrial appendage, the sealing portion 110 and the fixing portion 120 are assembled to obtain the occlusion device 100 that can better match the atrial appendage of the user. Undoubtedly, the occlusion device 100 in this embodiment has better adaptability.

In addition, since the occlusion device 100 is assembled, when the same quantity of sealing portions 110 and fixing portions 120 as the prior art are manufactured, the quantity of occlusion devices 100 that are obtained by combining the sealing portions 110 and the fixing portions 120 is greatly increased. For example, sealing portions 110 and fixing portions 120 which have M sets of sizes are manufactured. The operator can implement occlusion devices 100 of M*M specifications by preparing fixing portions 120 and sealing portions 110 of corresponding specifications, while there are only M sets of occlusion devices 100 in the prior art. This greatly improves the specification selection for the occlusion device 100. Still further, by using the occlusion device 100 of this embodiment, it is possible to produce different quantities of sealing portions 110 and fixing portions 120 which have different sizes to satisfy different combinations (such as sealing portions 110 with M sets of sizes and fixing portions 120 with N sets of sizes), thus eliminating one-to-one correspondence production of sealing portions 110 and fixing portions 120 according to batches, and improving production convenience.

In another embodiment, the sealing portion 110 and the fixing portion 120 are connected by an intermediate member. Specifically, the fixing portion 120 can be mounted and assembled to the intermediate member, and the sealing portion 110 can be mounted and assembled to the intermediate member, thereby achieving complete mounting of the occlusion device 100.

In another embodiment, the fixing portion 120 and the sealing portion 110 can rotate relative to each other.

In this embodiment, a first connection portion 130 is arranged at the distal end of the sealing portion 110. The first connection portion 130 can be fixed to a binding part of the braided mesh of the sealing portion 110 (for example, to a sleeve position of the sealing portion 110 by welding, threaded connection, etc.), or used as the binding part of the braided mesh of the sealing portion 110.

A second connection portion 140 is arranged at the proximal end of the fixing portion 120. The second connection portion 140 can be fixed to a binding part of the braided mesh of the fixing portion 120 (by welding, threaded connection, etc.), or used as a binding part of the support member 121 of the fixing portion 120.

The first connection portion 130 and the second connection portion 140 can be assembled. Considering that the occlusion device 100 formed by assembling is an implant, to lower the risk of detachment, in this embodiment, the first connection portion 130 and the second connection portion 140 cannot be disassembled after being assembled, that is, the fixing portion 120 and the sealing portion 110 are separate pieces that can be assembled, but cannot be disassembled after being assembled. In addition, it should be further noted that due to the particularity of the occlusion device 100 in this embodiment, the occlusion device 100 does not belong to a product type that can be reused or disassembled many times. In fact, disassembling the occlusion device 100 and reassembling it can easily affect the lives of the connection positions, and the increased risk far exceeds the need of cost saving. Since a margin needs to be reserved to disassemble the structure, the connection strength decreases. In this embodiment, it is preferred that the first connection portion 130 and the second connection portion 140 can be assembled, but cannot be disassembled. In this embodiment, this state is explained as follows: the portions can be assembled to form a stable connection. The stable connection only expresses that assembled objects are not detachable from each other, rather than expressing that the objects are relatively fixed and stationary each other. In fact, in other embodiments, the first connection portion 130 and the second connection portion 140 can still rotate relative to each other after being assembled. This still falls within the range of the stable connection.

In this embodiment, the first connection portion 130 includes at least one positioning member 131 distributed in a circumferential direction, and a connector 132 that extends out in the axial direction. The connector 132 includes a main body 1321 and a stop piece 1322 which are distributed in an extended manner from a proximal end to a distal end; and the diameter of the stop piece 1322 is greater than the diameter of the main body 1321.

In this embodiment, the second connection portion 140 includes a hollow tube body structure 141. The second connection portion 140 includes at least two stop sheets 142 that obliquely extend from the tube body structure 141 towards an inner side and the distal end and are oppositely arranged in a circumferential direction, thus forming a clip structure. Since the stop sheets 142 extend towards the inner side, the stop sheets 142 cannot be exposed outside and in contact with a sheath or hook the support member 121. For the tube body structure 141, the inner diameter of the tube body structure 141 is greater than an outer diameter of the connector 132, so that the connector 132 of the first connection portion 130 can extend into the tube body structure 141.

For the stop sheets 142, the diameter of an incomplete circle formed by distal ends (i.e. free ends) of at least two stop sheets 142 oppositely arranged in the circumferential direction is less than the outer diameter of each stop sheet 142. In other words, the inner diameter of a channel enclosed by free ends of the plurality of stop sheets 142 is less than the outer diameter of each stop sheet 142.

In another embodiment, the diameter of the stop piece 1322 gradually decreases from the proximal end to the distal end, thus allowing the stop piece 1322 to pass through the stop sheets 142 towards the distal end.

In this embodiment, preferably, a step surface is arranged at a connection position or a transition region between the stop piece 1322 and the main body 1321, so as to resist against the free ends of the stop sheets 142.

In this embodiment, at least one positioning slot 142 that matches the positioning member 131 is provided in the proximal end of the tube body structure 141. After the positioning member 131 is inserted into the positioning slot 142 in the axial direction, the first connection portion 130 is limited by both the positioning member 131 and the positioning slot 142 relative to the second connection portion 140, so that the first connection portion and the second connection portion cannot rotate freely. In one aspect, the positioning member 131 and the positioning slot 142 are distributed in the circumferential direction. A plurality of positioning members 131 and a plurality of positioning slots 142 match each other in a one-to-one manner, so that the first connection portion 130 and the second connection portion 140 are located at the same axis. This maintains the coaxiality between the fixing portion 120 and the sealing portion 110. On the other hand, more importantly, since the first connection portion 130 and the second connection portion 140 are assembled during use and serve as connection portions of the sealing portion 110 and the fixing portion 120, if the first connection portion 130 and the second connection portion 140 can rotate freely, it easily leads to loosening of the connection between the first connection portion 130 and the second connection portion 140 and the gradual enlargement of a gap, and detachment can easily occur. Therefore, the connection between the first connection portion 130 and the second connection portion 140 should be as stable and tight as possible. Objectively, it is also possible to design the fixing portion 120 to be rotatable relative to the sealing portion 110. However, for this embodiment, rotating portions of the sealing portion 110 and the fixing portion 120 should avoid the connection position between the first connection portion 130 and the second connection portion 140.

Further, due to the design of the positioning member 131 and the positioning slot 142, the sealing portion 110 can only be assembled with the fixing portion 120 at a specific angle. In this embodiment, the fixing portion 120 needs to be connected to the sealing portion 110 in the axial direction, and the positioning member 131 remains being clamped in the positioning slot 142 after being mounted.

In another embodiment, the positioning member 131 and the positioning slot 142 can also adapt to some special cases in which the fixing portion 120 and the sealing portion 110 need to be mounted at a specific angle. The positioning member 131 is obliquely arranged in the circumferential direction, and the positioning slot 142 matches the shape of the positioning member 131. Thus, the fixing portion 120 needs to be rotatably connected to the sealing portion 110 at the specific angle. The occlusion device formed by this combination has higher tightness, and the assembled positioning member 131 and positioning slot 142 can also assist in preventing relative movement between the fixing portion 120 and sealing portion 110 to an extent, thereby improving stability of the occlusion device.

In another embodiment, the inner diameter of the proximal end of the tube body structure 141 is greater than the inner diameter of the distal end. That is, the tube body structure 141 is configured to be flared, so that it is smoother to combine the first connection portion 130 with the second connection portion 140. It is more worth mentioning that when the proximal end of the tube body structure 141 is flared, edges of both the sealing portion 110 and the fixing portion 120 abut against an edge of a sheath during delivery inside the sheath, as long as the outer diameter of the tube body structure 141 is less than the inner diameter of the sheath. Due to the supporting performed by the sealing portion 110 and the fixing portion 120, the tube body structure 141 is basically located in the middle of the sheath, so that the delivery process cannot be affected by an increase in the diameter. In addition, when the occlusion device is withdrawn from the sheath, the distal end of the tube body structure 141 first leaves the sheath. The process of the tube body structure 141 leaving the sheath is smoother due to the guidance of its distal end.

In another embodiment, the positioning slot 142 does not extend through a side wall of the tube body structure 141. Thus, after the plurality of positioning members 131 respectively resist against the corresponding positioning slots 142, radial and circumferential positions of the first connection portion 130 and the second connection portion 140 have already been fixed.

In another embodiment, the second connection portion 140 is arranged at the distal end of the sealing portion 110, and the first connection portion 130 is arranged at the proximal end of the fixing portion 120.

It should be noted that the sealing portion 110 and the fixing portion 120 of the occlusion device 100 in this embodiment and the related embodiments are relatively independent before use, and they are assembled only during use. Generally, an operator holds the sealing portion 110 and the fixing portion 120 separately to achieve assembly and splicing. However, since the occlusion device 100 is an implant and has a high requirement for size accuracy, the manual operation of the operator cannot meet the requirement for precise mounting and easily leads to skewing. In addition, the occlusion device 100 cannot deform many times or to a large extent, but manual assembly easily leads to significant deformation, which can easily shorten the life of the occlusion device 100. Meanwhile, the mounting force of the occlusion device cannot be uniformly and stably controlled, which can easily cause damage to the instrument. Furthermore, during bare-hand assembly, it is inconvenient to hold the occlusion device. The operation is cumbersome and cannot quickly and accurately ensure the coaxiality of a fixing portion 120 and a sealing portion 110, resulting in long assembly time. Assembly is usually performed during surgery or preparatory surgery, which affects duration of the surgery. Therefore, in order to ensure the reliability of implantation, corresponding fixtures need to be introduced.

It should be noted that the assembly process of the occlusion device 100 of this embodiment requires high coaxial accuracy, that is, the sealing portion 110 and the fixing portion 120 need to be well aligned (or in a specific angle). Generally, it is not possible to perform assembly after the occlusion device is implanted into a human body. That is, the manner of sequential implantation, i.e. the manner of completing assembly by first implanting the fixing portion 120 and then implanting the sealing portion 110, cannot be used. In fact, objectively, it is difficult to implement the manner of completing assembly by first implanting the fixing portion 120 and then implanting the sealing portion 110, because the component implanted first can move with the heart, and the contraction and relaxation of the heart are not along a straight line. The fixing portion 120 implanted first is inevitably skewed from the implantation position, and the sealing portion 110 implanted later almost cannot be normally assembled. For an intracardiac implantation surgery with high safety risk, this assembly method is too risky and can only be implemented theoretically.

Based on the entire implantation process, after the sizes of the sealing portion 110 and the fixing portion 120 of the occlusion device 100 are selected, the occlusion device 100 is assembled and sheathed, and normal subsequent operations are performed. In the traditional sheathing process of the occlusion device 100, the occlusion device is first put into a loader and is then pushed into the sheath through the loader. Thus, there are two methods for assembling the occlusion device 100 to meet different surgical needs:
1. After the assembly is completed by using a fixture, the occlusion device 100 is put into the loader, and then the occlusion device 100 is pushed into the sheath through a push rod to achieve sheathing. The occlusion device 100 is assembled before loading.
2. The sealing portion 110 and the fixing portion 120 of the occlusion device 100 are at least partially put into a fixture that has a loading function, and then the assembly of the occlusion device 100 is completed while loading is implemented. Then, the occlusion device 100 is pushed into the sheath through a push rod to achieve sheathing. The occlusion device 100 is assembled and loaded simultaneously.

As mentioned earlier, the occlusion device 100 cannot deform many times or to a significant extent. Compression from the natural state to enter the loader and the subsequent sheathing process involve a significant deformation state, which cannot be avoided during the sheathing. When secondary release is required after the occlusion device is withdrawn from the sheath, the occlusion device needs to be retracted into the sheath and released, so it undergoes significant deformation. Generally, after the occlusion device 100 undergoes four to six significant deformations, its physical properties and service life may be affected. Although not all the physical properties and service life of the occlusion device 100 may be affected, due to a significantly increased risk, it is generally considered that the occlusion device 100 no longer meets implantation requirements and will be scrapped. Therefore, during the assembly, it is not allowed to compress the sealing portion 110 and the fixing portion 120 of the occlusion device 100 significantly in steps other than the sheathing process.

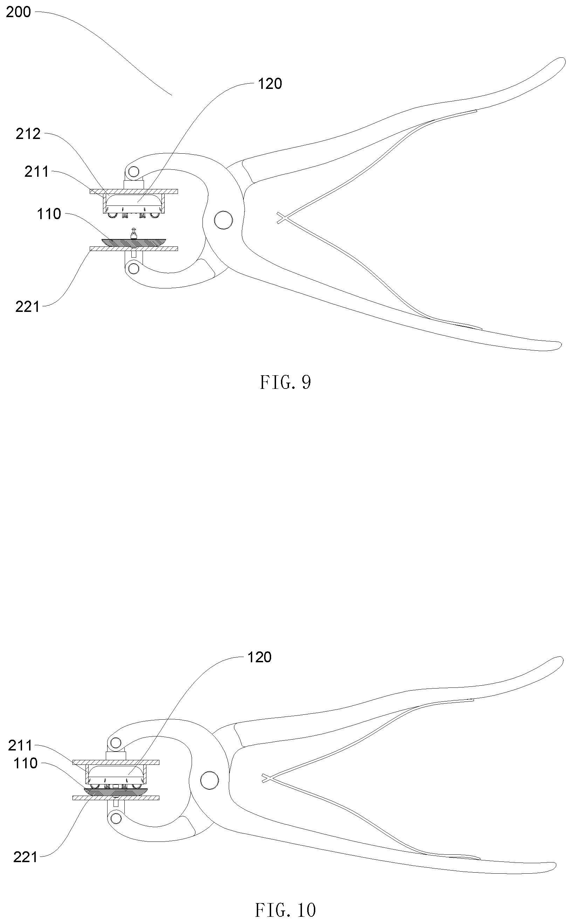

For assembly using method 1 above, as shown in FIG. 8 to FIG. 10, FIG. 8 is a schematic structural diagram of a first fixture for assembly of an occlusion device in one embodiment of the present application; FIG. 9 is a schematic diagram of a first state of a first fixture for assembly of an occlusion device in one embodiment of the present application; and FIG. 10 is a schematic diagram of a second state of a first fixture for assembly of an occlusion device in one embodiment of the present application. In the first state, the first fixture 200 respectively arranges the fixing portion 120 and the sealing portion 110 into corresponding fixtures, and in the second state, the first fixture 200 drives the fixing portion 120 and the sealing portion 110 to achieve assembly.

The first fixture 200 includes a first clamp 210 and a second clamp 220. The first clamp 210 and the second clamp 220 are opposite to each other. The first clamp 210 is provided with an annular enclosure 211 facing the second clamp 220, and the annular enclosure 211 is enclosed towards the second clamp 220 to form an accommodating cavity 212 with an opening. The accommodating cavity 212 is configured to accommodate the fixing portion 120. Preferably, the first clamp 210 is located vertically below the second clamp 220, so that the fixing portion 120 can be directly placed into the accommodating cavity 212 without falling out, thereby reducing contact deformation between the fixing portion 120 and the accommodating cavity 212. In fact, since the assembly process of the fixing portion 120 and the sealing portion 110 is in a vertical direction, the fixing portion 120 does not need to completely abut against or resist against the annular enclosure 211, as long as it ensures that the fixing portion directly faces the sealing portion 110 during the assembly.

If it is necessary to further improve the mounting accuracy or efficiency, the fixing portion 120 can be fixed to the accommodating cavity 212 with a small degree of deformation. An inner diameter of the accommodating cavity 212 is slightly less than an outer diameter of the fixing portion 120 (within 2 mm of the outer diameter of the fixing portion 120), thereby fixing the fixing portion 120.

In another embodiment, an inner surface of the accommodating cavity 212 includes an anchoring layer (a flexible material with elasticity, such as silicone and rubber), and an anchoring thorn structure of the fixing portion 120 can be anchored on the anchoring layer to achieve better fixation, without causing damage to the fixing portion 120.

In another embodiment, an anchoring layer is detachably sleeved on an inner side of the accommodating cavity 212, and an anchoring thorn structure of the fixing portion 120 can be anchored on the anchoring layer to achieve better fixation. The anchoring layer can be pushed out from the accommodating cavity 212 to be separated from the first clamp 210, making it easier for later removal.

In another embodiment, the annular enclosure 211 of the accommodating cavity 212 is variable in diameter, allowing it to adapt to sizes of various fixing portions 120. The second clamp 220 is provided with a platform 221 facing the first clamp 210. A bolt head (not shown in the figure) is arranged at a center position of the platform 221. The bolt head can be screwed into the proximal end of the sealing portion 110, so that the sealing portion 110 can be fixed on one side of the second clamp 220 that faces the first clamp 210. The platform 221 can rotate relative to its own central axis, making it easy to fix and release the sealing portion 110.

The first fixture 200 further includes a first arm 231 and a second arm 232 which are respectively connected to the first clamp 210 and the second clamp 220. The first arm 231 and the second arm 232 rotate relative to each other around a common pivot 233, thereby driving the first clamp 210 and the second clamp 220 to approach each other and move away from each other. The first clamp 210 and the second clamp 220 are located on one side of the pivot 233, and operating portions of the first arm 231 and the second arm 232 are on another side of the pivot 233. An elastic structure 240 is arranged between their operating portions. When an external force is applied to the operating portions of the first arm 231 and the second arm 232 to make them approach each other, the elastic structure 240 is compressed, and the first arm 231 and the second arm 232 approach each other, thereby making the first clamp 210 approach the second clamp 220 to achieve the assembly of the occlusion device. When the applied external force disappears, the elastic structure 240 returns to its original shape. Due to the completion of the assembly of the fixing portion 120 and the sealing portion 110, and threaded fixation of the sealing portion 110 and the platform 221, the restoration of the elastic structure 240 can drive the fixing portion 120 to be directly separated from the accommodating cavity 212, making it easier to remove the occlusion device.

In this embodiment, at least two groups of coaxial limiting members are arranged at peripheries of the first clamp 210 and the second clamp 220. Specifically, a plurality of vertically distributed columnar members are arranged at the periphery of the first clamp 210, and a plurality of fitting holes corresponding to columnar members are formed in the periphery of the second clamp 220. The columnar members move inside the fitting holes to form the coaxial limiting members, thereby limiting the coaxial accuracy of the first clamp 210 and the second clamp 220.

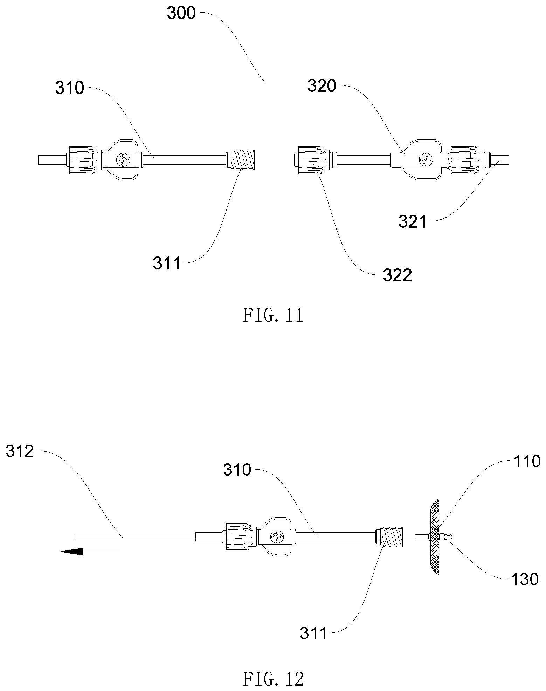

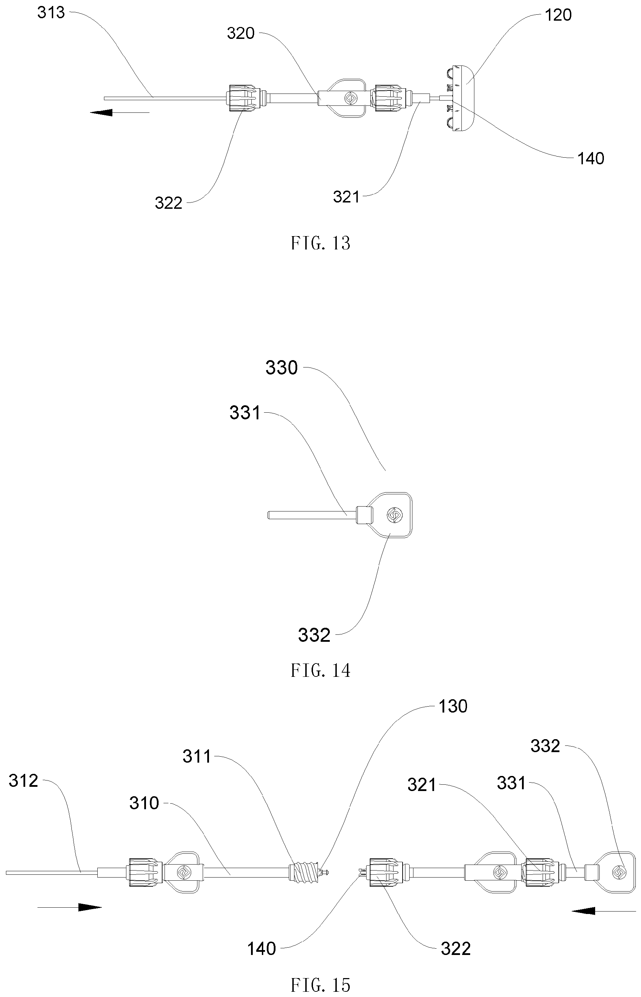

For assembly using method 2 above, referring to FIG. 11 to FIG. 15, FIG. 11 is a schematic structural diagram of a second fixture for assembly of an occlusion device in one embodiment of the present application; FIG. 12 is a schematic diagram of operation of one clamp of a second fixture for assembly of an occlusion device in one embodiment of the present application; FIG. 13 is a schematic diagram of operation of another clamp of a second fixture for assembly of an occlusion device in one embodiment of the present application; FIG. 14 is a schematic structural diagram of a blocking member in an embodiment of the present application; and FIG. 15 is a schematic diagram of operation of a second fixture for assembly of an occlusion device in one embodiment of the present application.

The second fixture 300 includes a group of tubular clamps with the same inner diameters, including a third clamp 310 and a fourth clamp 320. After the third clamp 310 and the fourth clamp 320 are combined, the second fixture 300 can also serve as a loader. That is, after the occlusion device is assembled through the second fixture 300, an operation for putting the occlusion device into the loader for sheathing is also implemented.

Specifically, one side of the third clamp 310 includes a first inlet 311. The first inlet 311 serves as an entrance for the sealing portion 110. Another side of the third clamp 310 extends into a first connection member 312. The first connection member 312 passes through an inner side of the third clamp 310 and extends out of the first inlet 311. A free end of the first connection member 312 includes a threaded structure, and the first connection member 312 is fixed to the center of the proximal end of the sealing portion 110 through the threaded structure, thereby fixing the sealing portion 110 to the first connection member 312. Based on this, the first connection member 312 is pulled to the left as illustrated in the diagram to drive the sealing portion 110 to be received into the third clamp 310, while the first connection portion 130 of the sealing portion 110 is located on the right side as illustrated in the diagram. As the entire sealing portion 110 is received into the third clamp 310, the sealing portion 110 itself is stretched and is in contact with an inner wall of the third clamp 310 in the circumferential direction for supporting each other to cause the first connection portion 130, which is located in an axis of the sealing portion 110, to be stably positioned in the axis. That is, this has good alignment.

Similarly, the fourth clamp 320 includes a second inlet 321 and a second outlet 322. The second inlet 321 serves as an entrance for the fixing portion 120, and another side of the fourth clamp 320 extends into a second connection member 313. The second connection member 313 passes through an inner side of the fourth clamp 320 and is fixedly connected to the fixing portion 120. It should be noted that the second connection member 313 is preferably a pull wire which is fixed by being wound around a hole or a cavity of the second connection portion 140 of the fixing portion 120, or being threaded around a central region of a rod of the fixing portion 120.

In another embodiment, part of a threaded ring is arranged on an inner side of the second connection portion 140 of the fixing portion 120, and the second connection member 313 is also a structure similar to a steel cable for pushing, which can be stably and reliably connected to the second connection portion 140.

The second connection member 313 is pulled to the left as illustrated in the diagram to drive the fixing portion 120 to be received into the fourth clamp 320, while the second connection portion 140 of the fixing portion 120 is located on the left side as illustrated in the diagram. As the entire fixing portion 120 is received into the fourth clamp 320, the fixing portion 120 itself is stretched and is in contact with an inner wall of the fourth clamp 320 in the circumferential direction for supporting each other to cause the second connection portion 140, which is located in an axis of the fixing portion 120, to be stably positioned in the axis. That is, this has good alignment.

It should be noted that after the storage of the fixing portion 120 is completed, the second connection member 313 of the fourth clamp 320 needs to be released from the fixing portion 120 and withdrawn.

Further, to ensure that the second connection portion 140 of the fixing portion 120 extends out of the second outlet 322 and is limited, a blocking member 330 is introduced. The blocking member 330 includes a tube 331 with an inner diameter slightly less than the inner diameter of the fourth clamp 320 and an operating portion 332. Inserting the tube 331 of the blocking member 330 into the fourth clamp 320 from the second inlet 321 and maintaining the position of the operating portion 332 can change the position of the distal end of the fixing portion 120 that has already been stored, and can also restrict movement of the fixing portion 120 towards the second inlet 321.

For the third clamp 310, a blocking member 330 can also be used. However, the first connection member 312 preferably employs a steel cable for delivery because the sealing portion 110 itself has reserved a connection structure for the steel cable for delivery, and the steel cable itself has strength. In this case, the steel cable itself can achieve both positional adjustment and limitation, which achieves the same effect.

After the storage of the sealing portion 110 and the fixing portion 120 is completed, the third clamp 310 and the fourth clamp 320 need to be spliced. In this case, the first inlet 311 of the third clamp 310 and the second outlet 322 of the fourth clamp 320 are adjacent to each other. The first connection portion 130 of the sealing portion 110 extends out from the first inlet 311, and the second connection portion 140 of the fixing portion 120 extends out from the second outlet 322. The first inlet 311 of the third clamp 310 and the second outlet 322 of the fourth clamp 320 have a mutually matching threaded connection structure. When the first inlet 311 of the third clamp 310 and the second outlet 322 of the fourth clamp 320 are spliced, it drives the sealing portion 110 and the fixing portion 120 to be assembled. Due to the good coaxiality between the first inlet 311 of the third clamp 310 and the second outlet 322 of the fourth clamp 320, the assembly process of the sealing portion 110 and the fixing portion 120 also has good coaxiality, so that skewing is almost unlikely to occur. Meanwhile, the third clamp 310 and the fourth clamp 320 which have been assembled can also function as loaders, so that one operating step can be saved, thus greatly improving working efficiency.

In another embodiment, the length of the fourth clamp 320 can be optimized, so that the second connection portion 140 of the fixing portion 120 slightly exceeds the fourth clamp 320 after being stored, and an operator can limit the fixing portion 120 by simply blocking the second inlet 321 with a hand. It should be noted that the various technical features of the embodiments described above can be combined arbitrarily and can also be applied simultaneously to various types of left atrial appendage occlusion devices mentioned above and left atrial appendage occlusion devices with similar structures. For the sake of brevity, all possible combinations of all the technical features in the above embodiments are not described. However, these technical features shall all be considered to fall within the scope of this specification as long as there is no contradiction in their combinations.

### Embodiment 2

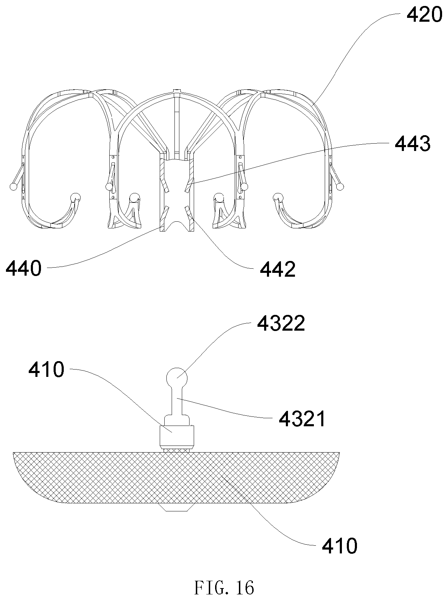

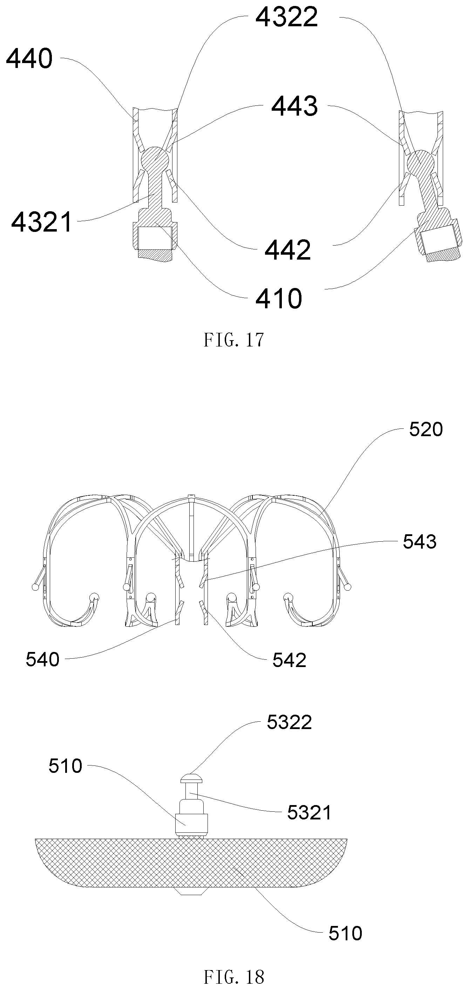

This embodiment is further optimized based on Embodiment 1. A specific difference is reflected in the connection position between the fixing portion and the sealing portion. As shown in FIG. 16 to FIG. 17, FIG. 16 is a schematic structural diagram of an occlusion device in Embodiment 2 of the present application, and FIG. 17 is a schematic diagram of connection of a fixing portion and a sealing portion of an occlusion device in Embodiment 2 of the present application. Both the first connection portion 430 of the sealing portion 410 and the second connection portion 440 of the fixing portion 420 are improved. The first connection portion 430 still includes a connector 432 that extends out in an axial direction. The connector 432 includes a main body 4321 and a stop piece 4322 which are distributed in an extended manner from the proximal end to the distal end. The diameter of the stop piece 4322 is greater than a diameter of the main body 4321. Additionally, the stop piece 4322 is a ball head. Thus, after assembling is completed, the stop piece 4322 can rotate in a plurality of directions along surfaces of the stop sheets 442 after crossing the stop sheets 442.

It should be noted that the rotation in this embodiment further includes deflection in a radial direction, that is, the sealing portion 410 can rotate in a plurality of directions in the radial direction relative to the fixing portion 420. Therefore, in addition to the stop piece 4322 being able to rotate, a space for rotation also needs to be reserved for the main body 4321 linked. Therefore, the second connection portion 440 is provided with a plurality of avoidance slots (not shown in the figure) that keep extending in the axial direction to penetrate through the proximal end, and a width of each avoidance slot is greater than the diameter of the main body 4321. Therefore, the main body 4321 can enter or pass through the avoidance slots to implement overall rotation. Meanwhile, only when positions of the avoidance slots are provided, the main body 4321 can rotate significantly and freely along the positions, which can limit a specific direction of the radial rotation of the sealing portion 410 relative to the fixing portion 420.

However, it should be noted that in the absence of the avoidance slots, since the diameter of the stop piece 4322 is greater than the diameter of the main body 4321, a gap exists between the main body 4321 and the second connection portion 440. Therefore, the main body 4321 can also rotate radially in a small angle within the second connection portion 440. The design of the avoidance slots is to meet a need of the sealing portion 410 to significantly radially rotate relative to the fixing portion 420.

In this embodiment, to avoid the risk of separation between the sealing portion 410 and the fixing portion 420 due to rotation, that is, to make the fixing portion 420 and the sealing portion 410 connected more stably, a group of stop sheets 443 that obliquely extend towards the proximal end are added at the distal end of the second connection portion 440. That is, extension directions of the stop sheets 443 and extension directions of the stop sheets 442 are opposite in the axial directions, and distances between the free ends of the stop sheets 443 and the free ends of the stop sheets 442 are less than or equal to the diameter of the stop piece 4322. After the stop piece 4322 crosses the stop sheets 442 from the proximal end to the distal end, the stop piece 4322 is pressed by the stop sheets 442 and the stop sheets 443, to keep the stop piece 4322 in the middle of the second connection portion 440, thereby causing the stop piece 4322 to rotate smoothly.

### Embodiment 3

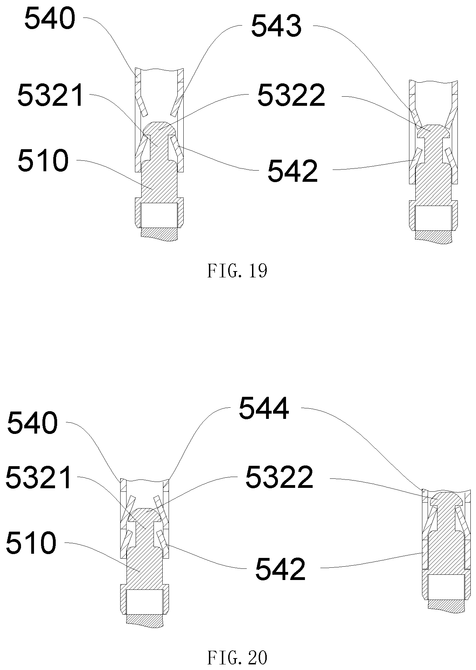

This embodiment is further optimized based on Embodiment 1. A specific difference is reflected in the connection position between the fixing portion and the sealing portion. As shown in FIG. 18 to FIG. 19, FIG. 18 is a schematic structural diagram of an occlusion device in Embodiment 3 of the present application, and FIG. 19 is a schematic diagram of connection of a fixing portion and a sealing portion of an occlusion device in Embodiment 3 of the present application. Both the first connection portion 530 of the sealing portion 510 and the second connection portion 540 of the fixing portion 520 are improved. The first connection portion 530 still includes a connector 532 that extends out in an axial direction. The connector 532 includes a main body 5321 and a stop piece 5322 which are distributed in an extended manner from the proximal end to the distal end. The diameter of the stop piece 5322 is greater than a diameter of the main body 5321 and is also greater than the diameter of an opening enclosed by the stop sheets 542 of the second connection portion 540 in the natural state. Thus, after assembling is completed and the stop piece 5322 crosses the stop sheets 542, the stop piece 5322 cannot be separated from surfaces of the stop sheets 542 along the proximal end, thus maintaining the connection between the sealing portion 510 and the fixing portion 520.

Additionally, distal end stop sheets 543 are arranged at the distal end of the second connection portion 540. The distal end stop sheets 543 obliquely extend towards the proximal end. Extension directions of the distal end stop sheets 543 and extension directions of the stop sheets 542 are opposite in the axial direction, and distances between the free ends of the stop sheets 543 and the free ends of the stop sheets 542 are greater than an axial width of the stop piece 5322. Therefore, after the stop piece 5322 crosses the stop sheets 542 from the proximal end to the distal end, the stop piece 5322 can move in the axial direction between the distal end stop sheets 543 and the stop sheets 542, so that relative axial movement can occur between the sealing portion 510 and the fixing portion 520, that is, the sealing portion 510 and the fixing portion 520 are movable in the axial direction. Therefore, when the occlusion device is implanted, a space for movement is provided for the sealing portion 510 and the fixing portion 520, so that it allows the sealing portion 510 and the fixing portion 520 to be staggered at the connection position. Since it is almost impossible for the fixing portion 520 and the sealing portion 510 to be located on the same axis during the implantation of the occlusion device, stress concentration is almost inevitable at the connection position between the sealing portion 510 and the fixing portion 520. The design of this embodiment can eliminate the stress concentration caused by the coaxial design of the sealing portion 510 and the fixing portion 520 to a maximum extent.

In another embodiment, referring to FIG. 20, FIG. 20 is a schematic diagram of connection of a fixing portion and a sealing portion of an occlusion device in Embodiment 3 of the present application. In this embodiment, the second connection portion 540 includes at least one group of distal end stop sheets 544. Extension directions of the distal end stop sheets 544 and extension directions of the stop sheets 542 in the axial direction are the same. That is, the distal end stop sheets 544 obliquely extend towards the distal end. Therefore, after crossing the stop sheets 542 from the proximal end to the distal end, the stop piece 5322 can further cross the distal end stop sheets 544. This further shortens the distance between the fixing portion 520 and the sealing portion 510. It should be noted that, in one aspect, during implantation in this embodiment, the fixing portion 520 is released first, and the sealing portion 510 is released later. If the stop piece 5322 is pressed to deform after the implantation, the stop piece further crosses the distal end stop sheets 544 from the proximal end to the distal end, thus shortening the distance between the fixing portion 520 and the sealing portion 510. Since the position of the fixing portion 520 remains unchanged, the sealing portion 510 can be further tightened, which in turn promotes tighter occlusion. In another aspect, since the fixing portion 520 is released first and the sealing portion 510 is released later, and the fixing portion 520 has already been anchored after being released, an operator can select a desired distance between the fixing portion 520 and the sealing portion 510. If the distance needs to be shortened, the sealing portion 510 can be pushed further to move towards the distal end to continue crossing the distal end stop sheets 544, so as to meet the requirements.

In another embodiment, the second connecting portion 540 is provided with a plurality of groups of distal end stop sheets 544 in the axial direction, thereby implementing multi-level adjustment on the distance between the fixing portion 520 and the sealing portion 510. Further, to implement adjustment in more levels, all the distal end stop sheets 544 are located at different positions in the circumferential direction.

The foregoing embodiments merely express several implementations of the present application. The descriptions thereof are relatively specific and detailed, but are not understood as limitations on the scope of the present application. For a person of ordinary skill in the art, several transformations and improvements can be made without departing from the idea of present application. These transformations and improvements belong to the protection scope of present application. Therefore, the protection scope of the patent of the present application shall be subject to the appended claims.

## Claims

1. An occlusion device, comprising a fixing portion and a sealing portion, wherein a first connection portion is arranged at a proximal end of the fixing portion, and a second connection portion is arranged at a distal end of the sealing portion; the occlusion device has an initial state and an assembled state; in the initial state, the first connection portion and the second connection portion are separated from each other; and in the assembled state, the first connection portion is connected to the second connection portion to connect the fixing portion to the sealing portion.

2. The occlusion device according to claim 1, wherein the fixing portion and the sealing portion are stably connected after being assembled.

3. The occlusion device according to claim 1, wherein the first connection portion comprises a connector that extends out in an axial direction; the connector comprises a main body and a stop piece which are distributed in an extended manner from a proximal end to a distal end; and the diameter of the stop piece is greater than the diameter of the main body.

4. The occlusion device according to claim 3, wherein the second connection portion comprises a hollow tube body structure; and the second connection portion comprises at least two stop sheets that obliquely extend out from the tube body structure towards an inner side and the distal end and are oppositely arranged in a circumferential direction.

5. The occlusion device according to claim 4, wherein the inner diameter of the tube body structure is greater than the outer diameter of the connector; and the inner diameter of a channel enclosed by free ends of the plurality of stop sheets is less than the outer diameter of each stop sheet.

6. The occlusion device according to claim 3, wherein the diameter of the stop piece gradually decreases from the proximal end to the distal end.

7. The occlusion device according to claim 3, wherein a step surface is arranged at a connection position or a transition region between the stop piece and the main body.

8. The occlusion device according to claim 1, wherein the first connection portion comprises at least one positioning member distributed in a circumferential direction; and at least one positioning slot that matches the positioning member is arranged at a proximal end of the second connection portion.

9. The occlusion device according to claim 1, wherein a proximal end surface or a distal end surface of the sealing portion comprises a straight section and a curved section; the curved section is located on an outer side of the straight section; and an edge of the curved section is located at a distal end of the straight section.

10. The occlusion device according to claim 9, wherein in a natural state, the length the curved section from a proximal end to a distal end is 1 to 5 mm.
